# EUROPÄISCHE PATENTSCHRIFT

(11) **EP 3 052 467 B1**
(45) Veröffentlichungstag und Bekanntmachung des Hinweises auf die Patenterteilung: **18.12.2019**
(21) Anmeldenummer: 14777311.3
(22) Anmeldetag: 29.09.2014
(51) Int. Cl.: C07C 67/08, C07C 69/54

(54) **VERFAHREN ZUR HERSTELLUNG VON (METH)ACRYLIERTEN BENZOPHENONEN**
METHOD FOR PRODUCING (METH)ACRYLATED BENZOPHENONES
PROCÉDÉ DE FABRICATION DE BENZOPHÉNONES (MÉTH)ACRYLÉES

(30) Priorität: 04.10.2013 DE 102013220127
(43) Veröffentlichungstag der Anmeldung: 10.08.2016
(73) Patentinhaber: Evonik Operations GmbH, 45128 Essen (DE)
(72) Erfinder: KLESSE, Wolfgang, 55127 Mainz (DE); KNEBEL, Joachim, 64665 Alsbach-Hähnlein (DE); SAAL, Doris, 64625 Bensheim (DE)
(74) Vertreter: Evonik Patent Association
(86) Internationale Anmeldenummer: PCT/EP2014/070787
(87) Internationale Veröffentlichungsnummer: WO 2015/049200

(56) Entgegenhaltungen:
- DE-A1-102008 054 611
- JP-A- 2003 261 506
- JP-A- 2003 261 556

## Beschreibung

Die Erfindung betrifft ein Verfahren zur Herstellung von Methacrylsäureestern von hydroxyfunktionellen Benzophenonen und deren Verwendung. Mit dem erfindungsgemäßen Verfahren lassen sich auf einfache Weise und in hoher Ausbeute methacrylierte Benzophenone in hoher Reinheit herstellen.

Im Stand der Technik ist ein Methacrylsäureanhydridverfahren zur Herstellung der oben genannten Ester beschrieben (JP 2003261506, Mitsubishi Rayon). Als Katalysator wird Triethylamin eingesetzt. Da das Amin mit der bei der Reaktion entstehenden Methacrylsäure ein Salz bildet, muss es äquimolar zum Benzophenon bemessen werden. Entsprechend erhält man äquimolare Salzmengen, die als Abfall entsorgt werden müssen. Das Verfahren ist daher wenig wirtschaftlich. Weitere Methoden des Standes der Technik sind die Umsetzung von Methacrylsäurechlorid mit hydroxyfunktionellem Benzophenon sowie die Umsetzung dieses Rohstoffs mit Glycidylmethacrylat. Beim Umgang mit Methacrylsäurechlorid müssen die korrosiven und ätzenden Eigenschaften beachtet werden. Bei Kontakt mit Wasser wird zudem HCl frei.

In der DE 1720603 wird ein Verfahren zur Herstellung von wässrigen Dispersionen leicht vernetzbarer Polymerisate beschrieben. Hierbei werden Acryl-und Methacrylsäureester mit lichtaktiven, olefinisch ungesättigten Monomeren, u.a. Benzophenonderivaten, copolymerisiert, ggf. unter Mitverwendung von lichtaktiven, nichtionogenen Emulgatoren.

Die EP 0346788 beschreibt ein Verfahren zur Herstellung strahlungsempfindlicher Carbamoylbenzo- und -acetophenone mit mindestens einer Methacrylat- bzw. Acrylatendgruppe. Dabei werden Isocyanatoalkyl(meth)acrylate mit Hydroxyacetophenonen oder Hydroxybenzophenonen mit einem basischen Katalysator umgesetzt. Hierbei muss unter Feuchtigkeitsausschluss gearbeitet werden. Zudem können nur getrocknete nicht nukleophile Lösungsmittel verwendet werden.

Die WO 2010/072479 beschreibt die Herstellung der oben genannten Ester in Gegenwart katalytischer Mengen Schwefelsäure. Der Katalysator muss nach beendeter Umsetzung mit Natronlauge neutralisiert und das Natriumsulfat abfiltriert werden.

Bei der salzkatalysierten Veresterung wird der zu veresternde Alkohol in Gegenwart kleiner Mengen eines Salzes der dem Anhydrid zugrunde liegenden Säure mit dem Carbonsäureanhydrid umgesetzt. Gemäß der DE 3601098 oder Autorenkollektiv, Organikum, 20. Aufl. 1999, S. 444-445 können so in Gegenwart von Acetanhydrid und Natriumacetat Zucker zu den Acetaten verestert werden. Jedoch werden große Mengen an Katalysator und/oder an Acylierungsmittel benötigt. Das dem 4-Hydroxybenzophenon ähnelnde Dithranol wird mit 20 % seiner molaren Menge an Anhydrid umgesetzt bzw. muss mit einem 3,3 fach molaren Überschuss Acetanhydrid in Gegenwart von Natriumacetat acetyliert werden (H. P. Faro u. a., Arch. Pharm. 313, 800 (1980)). Bei der Zuckerveresterung setzt man einen großen Überschuss an Acylierungsmittel ein (1,76 mol/mol).

Aufgabe der Erfindung war es daher, ein weiter vereinfachtes Herstellungsverfahren bereitzustellen, bei dem der Katalysator ohne zusätzliche Schritte wie z. B. eine Neutralisation abgetrennt werden kann und bei dem eine nur geringe Salzfracht mitgeführt werden muss.

Die Aufgabe wird gelöst durch ein Verfahren zur Herstellung von Benzophenon(meth)acrylaten, dadurch gekennzeichnet, dass Hydroxybenzophenon und (Meth)acrylsäureanhydrid in Gegenwart katalytischer Salzmengen ausgewählt aus der Gruppe Lithium- oder Kaliumacetate bzw. Li-, Na- oder K-(Meth)acrylate, bevorzugt Natriumacetat, umgesetzt werden, wobei das Stoffmengen-Verhältnis (Meth)acrylsäureanhydrid zu Hydroxybenzophenon 1,0:1,0 bis 1,6:1,0 ist.

Die Schreibweise (Meth)acrylat bedeutet hier sowohl Methacrylat, wie z. B. Methylmethacrylat, Ethylmethacrylat usw., als auch Acrylat, wie z. B. Methylacrylat, Ethylacrylat usw., sowie Mischungen aus beiden.

Überraschend wurde gefunden, dass mit dem erfindungsgemäßen Verfahren ein hoher Umsetzungsgrad erreicht werden kann. Das erfindungsgemäße Verfahren ist zudem durch eine lediglich geringe Salzfracht belastet. Das Salz kann leicht abgetrennt werden.

In einer besonderen Ausführungsform wird die als Nebenprodukt anfallende (Meth)acrylsäure bei einer anschließenden Polymerisation des Benzophenon-Monomeren als Comonomer mit verwendet. In einer anderen Ausführungsform wird die (Meth)acrylsäure zur Herstellung von neuem (Meth)acrylsäureanhydrid recycliert.

In einer bevorzugten Ausführungsform liegt das Stoffmengen-Verhältnis (Meth)acrylsäureanhydrid zu Hydroxybenzophenon im Bereich von 1,0:1,0 bis 1,6:1,0. In einer anderen Ausführungsform liegt das Verhältnis im Bereich von 1,1:1,0 bis 1,4:1,0, weiter bevorzugt im Bereich von 1,1:1,0 bis 1,2:1,0. Ganz besonders bevorzugt beträgt das Stoffmengen-Verhältnis (Meth)acrylsäureanhydrid zu Hydroxybenzophenon 1,1:1,0.

In einer Ausführungsform werden mehrere Hydroxybenzophenone eingesetzt. Besonders bevorzugt sind dabei Hydroxybenzophenone ausgewählt aus der Gruppe bestehend aus 4-, 3- oder 2-Hydroxybenzophenon bzw. deren Isomeren-oder andere Gemische sowie bifunktionelle Hydroxybenzophenone wie 2,3-, 2,4-oder 4,4'-Dihydroxybenzophenone bzw. deren Isomeren- oder andere Gemische.

In einer anderen Ausführungsform werden Gemische aus Methacrylsäureanhydrid und Acrylsäureanhydrid eingesetzt.

In einer besonderen Ausführungsform wird 4-Hydroxybenzophenon mit Methacrylsäureanhydrid zu 4-(Methacryloyloxy)benzophenon umgesetzt.

In einer bevorzugten Ausführungsform wird als Katalysator Natriumacetat eingesetzt. Ferner geeignet sind entsprechende Lithium- oder Kaliumacetate bzw. Li-, Na- oder K-(Meth)acrylate. Besonders bevorzugt wird Na-Methacrylat eingesetzt. In einer anderen Ausführungsform werden die genannten Salze aus den entsprechen Methanolaten bzw. aus deren Methanol-Lösungen während der Reaktion aus zugesetzter Essigsäure bzw. aus dem anwesenden (Meth)acrylsäureanhydrid in situ erzeugt. Alternativ können die entsprechenden Salze nach separater Herstellung der Reaktion zugesetzt werden.

In einer bevorzugten Ausführungsform wird der Katalysator in einer Menge von 0,2 bis 5,0 mol% bezogen auf die Stoffmenge des verwendeten (Meth)acrylsäureanhydrids eingesetzt. In einer anderen Ausführungsform wird der Katalysator in einer Menge von 0,3 bis 4,0 mol%, bevorzugt von 0,45 bis 2,5 mol%, besonders bevorzugt von 0,5 bis 2,3 mol% oder bis 0,7 mol% bezogen auf die Stoffmenge des verwendeten (Meth)acrylsäureanhydrids eingesetzt. In einer besonders bevorzugten Ausführungsform wird der Katalysator in einer Menge von 0,5 mol% bezogen auf die Stoffmenge des verwendeten (Meth)acrylsäureanhydrids eingesetzt.

Mit dem erfindungsgemäßen Verfahren lassen sich Benzophenon(meth)acrylate als Feststoff gewinnen. In einer bevorzugten Ausführungsform werden das/die Hydroxybenzophenon(e) mit dem/den (Meth)acrylsäureanhydrid(en) bei einer Temperatur im Bereich von 50 bis 120 °C, besonders bevorzugt im Bereich von 70 bis 110 °C, weiter bevorzugt im Bereich von 85 bis 95 °C oder 90 bis 100 °C, ganz besonders bevorzugt bei 90 °C umgesetzt.

Bevorzugt beträgt die Reaktionszeit, insbesondere bei 90 °C, 1 bis 13 h, besonders bevorzugt 1,5 bis 11 h, weiter bevorzugt 4 bis 8 h.

Die anschließende Aufbereitung des Rohmonomers erfolgt, ggf. nach der Hydrolyse des (Meth)acrylsäureanhydridüberschusses z. B. durch Zugabe von Methanol oder durch Einrühren in Wasser. So werden die wasserlöslichen Verunreinigungen gelöst und können problemlos abgetrennt werden. Das Benzophenon(meth)acrylat wird im Wasser ausgefällt und durch Filtration in fester Form isoliert.

Die in hoher Reinheit hergestellten Benzophenon(meth)acrylate können in Lösung mit Methylmethacrylat, n-Butylmethacrylat, i-Butylmethacrylat oder Styrol gelagert und weiter umgesetzt werden.

Mit dem erfindungsgemäßen Verfahren lassen sich ebenfalls Benzophenon(meth)acrylate in einer (Meth)acrylsäurealkylester-Lösung gewinnen. Weitere geeignete Lösungen sind Styrol-Lösungen. In einer bevorzugten Ausführungsform werden das/die Hydroxybenzophenon(e) zunächst mit dem/den (Meth)acrylsäureanhydrid(en), Katalysator und optional Stabilisatoren vermischt. Anschließend wird die gewünschte Reaktionstemperatur eingestellt. In einer bevorzugten Ausführungsform werden das/die Hydroxybenzophenon(e) mit dem/den (Meth)acrylsäureanhydrid(en) bei einer Temperatur im Bereich von 50 bis 120°C, besonders bevorzugt im Bereich von 70 bis 110°C, weiter bevorzugt im Bereich von 85 bis 95°C oder 90 bis 100°C, ganz besonders bevorzugt bei 90°C umgesetzt. Zur Erzeugung der genannten Lösung in (Meth)acrylsäurealkylestern wird nach erfolgter Umsetzung (Meth)acrylsäurealkylester bzw. ggf. Mischungen derselben zugefügt, um die gewünschte Konzentration an Benzophenon(meth)acrylat zu erhalten. Salze und Verunreinigungen werden danach über einen geeigneten Druckfilter abgetrennt.

Bevorzugt beträgt die Reaktionszeit, insbesondere bei 90 °C, 2 bis 8 h, besonders bevorzugt 3 bis 7 h, weiter bevorzugt 4 bis 8 h, insbesondere 4,5 bis 5,5 h.

Bevorzugt ist der (Meth)acrylsäurealkylester ausgewählt aus der Gruppe bestehend aus (Meth)acrylsäuremethylester, n-Butylmethacrylat oder i-Butylmethacrylat. Ferner eignen sich auch Hydroxyalkyl(meth)acrylate, inbesondere Hydroxyethyl(meth)acrylat bzw. Hydroxypropyl(meth)acrlat. Besonders bevorzugt ist Methylmethacrylat (MMA).

Gegenstand des Patentes ist daher auch die Herstellung einer Lösung von Benzophenon(meth)acrylaten in (Meth)acrylsäurealkylestern oder in Styrol. In einer bevorzugten Ausführungsform wird 4-(Methacryloyloxy)benzophenon in einer 30 %igen MMA-Lösung hergestellt.

Benzophenon(meth)acrylate können zur nachträglichen Photovernetzung von Polymeren durch Tages- oder UV-Licht sowie als polymere Photoinitiatoren verwendet werden.

Die Benzophenon(meth)acrylate können zudem als Monomere und/oder Comonomere für Polymerisationsreaktionen verwendet werden.

Die nachfolgenden Beispiele sollen die Erfindung näher erläutern, diese jedoch nicht auf die hierin offenbarten Merkmale beschränken.

### Beispiele

### Abkürzungen:

- MAAH: (Meth)acrylsäureanhydrid
- MeOH: Methanol
- MMA: Methylmethacrylat
- MAS: Methacrylsäure
- NM30-Lösung: Natriummethanolat, 30 %ig in Methanol, Fa. Evonik
- GC Fl%: Flächenprozente nach Gaschromatographie
- GC; Gew%: Gewichtsprozente nach Gaschromatographie
- HQME: Hydrochinonmonomethylether
- SZ: Säurezahl in mg KOH/g
- Topanol A: 2,4-Dimethyl-6-tert.butylphenol (Polymerisationsinhibitor)

Gaschromatographische Analytik: Gerät Agilent 7820A mit FID
Kapillarsäule DB5, 30 m lang, Durchmesser 0,25 mm, Schichtdicke 0,25 µm
Injektortemperatur 250°C, Detektortemperatur 300°C
Temperaturprogramm: 80°C für 2 min, dann mit 16°C /min aufheizen auf 300°C, diese Temperatur 6 min halten.

### Beispiel 1: Katalysator Natriummethacrylat in situ erzeugt

Apparatur: 2 l Vierhalsrundkolben mit mechanischer Rührung, Rückflusskühler, Lufteinleitung, Pt100-Temperaturfühler, Ölbad.
Ansatz: 1,5 mol (297,9 g) 4-Hydroxybenzophenon, 1,65 mol (270,3 g) MAAH, 0,0087 mol (1,6 g) NM30-Lösung, 399 mg 2,4-Dimethyl-6-tert-butylphenol (Topanol A);
Herstellung einer 30 %igen Lösung von 4-(Methacryloyloxy)benzophenon in MMA: Zugabe von insgesamt 772 g MMA
Theoretische Ausbeute an 4-(Methacryloyloxy)benzophenon: 399 g
Durchführung: 4-Hydroxybenzophenon wurde eingewogen, dazu wurden 126 g des später benötigten MMA zugegeben, es entstand eine gut rührbare Masse. MAAH, Stabilisator Topanol A und Katalysator NM30 wurden anschließend zugegeben, Luft eingeleitet und auf 90 °C erhitzt. Der Reaktionsverlauf wurde durch Gaschromatographie verfolgt (eine Probe (ca. 1,5 ml) genommen, mit ca. 5 ml Aceton verdünnt, filtriert). Nach insgesamt 5 h bei 90 °C wurde auf 60 °C abgekühlt, 30 g Methanol zur Umsetzung des verbliebenen MAAH zugegeben und 1 h bei 60 °C gerührt. Zum Ansatz wurde dann der Rest des MMA (646 g) gegeben, um eine 30 %ige Lösung herzustellen. Es wurde unter Rühren abgekühlt und über einen Druckfilter mit Filterschicht Seitz T1000 (Ø 14 cm) filtriert. Ausbeute: 1320,4 g

### Analysen:

Wassergehalt nach Karl Fischer: 0,03 %
Topanol A: 538 ppm

| | | |
|---|---|---|
| GC F1%: | 52,543 % | MMA |
| | 9,491 % | MAS |
| | 0,829 % | 4-Hydroxybenzophenon |
| | 0,701 % | 4-Acetoxybenzophenon |
| | 34,921 % | 4-(Methacryloyloxy)benzophenon |
| | | |
| GC; Gew.%: | MMA 57,9 % | |
| | MAS 10,9 % | |
| | 4-(Methacryloyloxy)benzophenon 28,5 % | |

Hazen-Farbzahl: 315
SZ: 69 = 10,58 % MAS

### Beispiel 2: Herstellung von 4-(Methacryloyloxy)benzophenon als Feststoff mit in situ erzeugtem Katalysator Natriummethacrylat.

Apparatur: siehe Beispiel 1
Ansatz: 4,0 mol (794,7 g) 4-Hydroxybenzophenon, 4,4 mol (723,7 g) MAAH, 0,0228 mol (4,2 g) NM30-Lösung, 1055 mg Topanol A;
Theoretische Ausbeute:4-(Methacryloyloxy)benzophenon: 1055,2 g
Durchführung: Der Ansatz wurde komplett eingewogen und dann auf 90 °C erhitzt. Nach 6 h Reaktionszeit betrug der 4-Hydroxybenzophenon-Anteil noch 2,2 % (GC-Analytik), es wurde 2 ml NM30-Lösung nachgegeben und weiter erhitzt. Nach insgesamt 11 h bei 90 °C wurde auf 60 °C abgekühlt und 80 g Methanol zur Veresterung des unumgesetzten MAAH zugegeben. Anschließend wurde 1 h bei 60 °C gerührt. Dann wurde der Ansatz unter Rühren (Metallflügelrührer, Rührmotor) im dünnen Strahl in 3 l Wasser gegossen. Es wurde 0,5 h gerührt, und die Ausfällung dann über eine Glasfilterfritte abgesaugt, noch einmal mit 2,0 l Wasser nachgewaschen ( 30 min im Becherglas mit einem Rührmotor mit Metallflügelrührer verrührt) und anschließend auf der Nutsche trocken gesaugt. Der Feststoff wurde anschließend bis zur Massekonstanz luftgetrocknet.
Ausbeute: 1042,3 g

### Analysen:

Wassergehalt (Karl Fischer): < 0,1 %
Topanol A: 808 ppm

| | | |
|---|---|---|
| GC: | 0,066 % | MAS |
| | 1,239 % | 4-Hydroxybenzophenon |
| | 1,785 % | 4-Acetoxybenzophenon |
| | 95,574 % | 4-(Methacryloyloxy)benzophenon |

Hazen-Farbzahl 20 %ig in Aceton: 154

### Beispiel 3: Herstellung von 4-(Methacryloyloxy)benzophenon als Feststoff mit separat hergestelltem Katalysator Natriummethacrylat.

Apparatur: siehe Beispiel 1
Ansatz: 0,5 mol (99,4 g) 4-Hydroxybenzophenon, 0,55 mol (88,3 g) MAAH, 0,0125 mol (1,35 g) Natriummethacrylat, 133 mg HQME, 133 mg Topanol A;
Theoretische Ausbeute an 4-(Methacryloyloxy)benzophenon: 133,15 g
Durchführung: Der Ansatz wurde komplett eingewogen und dann unter Lufteinleiten auf 90 °C erhitzt. Der Umsatz wurde mittels Gaschromatographie (GC) verfolgt. Zusammensetzung nach 1,5 h: 18,42 % MAS, 2,5 % MAAH, 0,83 % Hydroxybenzophenon, 75,84 % 4-(Methacryloyloxy) benzophenon. Es wurde auf 60 °C abgekühlt, Methanol (10 g) zugegeben und 1 h bei 60 °C gerührt.

Dann wurde der Ansatz unter Rühren (Metallflügelrührer, Rührmotor) im dünnen Strahl in 500 ml Wasser gegossen. Es wurde 0,5 h gerührt und die Ausfällung dann über eine Glasfilterfritte abgesaugt, noch zweimal mit je 500 ml Wasser nachgewaschen (je 15 min im Becherglas mit einem Rührmotor mit Metallflügelrührer gerührt) und anschließend auf der Nutsche trocken gesaugt. Der Feststoff wurde anschließend bis zur Massekonstanz luftgetrocknet. Ausbeute: 131,1 g

### Analysen:

Wassergehalt nach Karl Fischer: 0,21 %
HQME: 13 ppm
Topanol A: 1305 ppm

| | | |
|---|---|---|
| GC: | 0,012 % | MMA |
| | 0,331 % | MAS |
| | 1,573 % | 4-Hydroxybenzophenon |
| | 94,594 % | 4-(Methacryloyloxy)benzophenon |

Farbzahl 20 %ig in Aceton: 82

### Beispiel 4: Darstellung von 4-(Methacryloyloxy)benzophenon mit Katalysator Natriummethacrylat; Umsatzkontrolle

Apparatur: siehe Beispiel 1
Ansatz:0,5 mol (99,4 g) 4-Hydroxybenzophenon, 0,55 mol (88,3 g) MAAH, 0,003 mol (0,32 g) Natriummethacrylat, 133 mg HQME, 133 mg Topanol A;
Theoretische Ausbeute an 4-(Methacryloyloxy)benzophenon: 133,15 g
Durchführung: Der Ansatz wurde komplett eingewogen und dann auf 90 °C erhitzt. Der Umsatz wurde mittels GC verfolgt.

**Zusammensetzung in Gew%:**

| Reaktionszeit | MAS | MAAH | Hydroxybenzophenon | 4-(Methacryloyloxy)-benzophenon |
|---|---|---|---|---|
| 1 h | 16,69 | 6,47 | 4,66 | 69,72 |
| 2 h | 18,03 | 4,39 | 2,83 | 72,07 |
| 3 h | 18,95 | 3,46 | 1,21 | 73,96 |
| 4 h | 17,69 | 2,96 | 1,25 | 74,88 |

Es wurde auf 60 °C abgekühlt, Methanol (9,6 g) zur Veresterung des verbliebenen MAAH zugegeben und 1 h bei 60 °C gerührt. Dann wurde der Ansatz unter Rühren (Metallflügelrührer, Rührmotor) im dünnen Strahl in 500 ml Wasser gegossen. Es wurde 0,5 h gerührt und die Ausfällung dann über eine Glasfilterfritte abgesaugt, noch zweimal mit je 500 ml Wasser nachgewaschen (je ca. 15 min im Becherglas mit einem Rührmotor mit Metallflügelrührer gerührt) und anschließend auf der Nutsche trockengesaugt.

Der Feststoff wurde anschließend bis zur Massekonstanz luftgetrocknet.
Ausbeute: 136,7 g

### Analysen:

H₂O: 0,25 %
HQME: 15 ppm
Topanol A: 1530 ppm

| | | |
|---|---|---|
| GC: | 1,064 % | MAS |
| | 2,571 % | 4-Hydroxybenzophenon |
| | 92,754 % | 4-(Methacryloyloxy)benzophenon |

Farbzahl 20%ig in Aceton: 144

### Beispiel 5: Katalysator Natriumacetat

Apparatur: 2 l Vierhalsrundkolben mit mechanischem Rührer, Rückflusskühler, Lufteinleitrohr und Pt100-Temperaturfühler, Ölbad

### Ansatz:

0,5 mol 4-Hydroxybenzophenon, Reinheit 99,9% = 99,2 g
0,55 mol Methacrylsäureanhydrid, Reinheit 97,7% = 86,9 g
0,0125 mol Natriumacetat, wasserfrei = 1,025 g
Topanol A = 93,2 mg
Zur Veresterung des Methacrylsäureüberschusses: 9,6 g Methanol
Theoretische Ausbeute an 4-(Methacryloyloxy)benzophenon : 133,2 g
Durchführung: 4-Hydroxybenzophenon wurde eingewogen. Methacrylsäureanhydrid, Topanol A und Natriumacetat wurden anschließend zugegeben und dann unter Luftzufuhr auf 90°C erhitzt. Es wurde 6 h gerührt, dann auf 60°C abgekühlt und das Methanol zur Veresterung des verbliebenen Methacrylsäureanhydrids zugegeben. Es wurde 1 h weiter bei 60°C gerührt. Der Ansatz wurde dann unter Rühren in 500 ml H₂O eingetragen, 0,5 h gerührt, abgesaugt und noch einmal mit 500 ml Wasser im Becherglas gewaschen und dann auf der Nutsche trockengesaugt. Das Produkt wurde luftgetrocknet.
Ausbeute: 130 g 4-(Methacryloyloxy)benzophenon

### Analysen:

Wassergehalt : 0,36 % Topanol A: 1390 ppm
Hazen-Farbzahl (10% in Aceton): 16

| | | |
|---|---|---|
| GC-Fl%: | 0,011 % | MMA |
| | 0,650% | MAS |
| | 1,834 % | 4-Hydroxybenzophenon |
| | 2,928% | 4-Acetoxybenzophenon |
| | 91,560 % | 4-(Methacryloyloxy)benzophenon |

## Patentansprüche

1. Verfahren zur Herstellung von Benzophenon(meth)acrylaten, **dadurch gekennzeichnet, dass** Hydroxybenzophenon und (Meth)acrylsäureanhydrid in Gegenwart katalytischer Salzmengen, ausgewählt aus der Gruppe bestehend aus Natrium-, Lithium- oder Kaliumacetate bzw. Li-, Na- oder K-(Meth)acrylate, , umgesetzt werden,
wobei das Stoffmengen-Verhältnis (Meth)acrylsäureanhydrid zu Hydroxybenzophenon 1,0:1,0 bis 1,6:1,0 beträgt.

2. Verfahren nach Anspruch 1, wobei das Stoffmengen-Verhältnis im Bereich von 1,05:1,0 bis 1,5:1,0, bevorzugt im Bereich von 1,1:1,0 bis 1,4:1,0, weiter bevorzugt im Bereich von 1,1:1,0 bis 1,2:1,0 liegt.

3. Verfahren nach Anspruch 1 oder 2, wobei als Hydroxybenzophenon 4-Hydroxybenzophenon eingesetzt wird.

4. Verfahren nach einem der vorstehenden Ansprüche, wobei Methacrylsäureanhydrid oder Acrylsäureanhydrid eingesetzt wird.

5. Verfahren nach einem der vorstehenden Ansprüche, wobei als Katalysator Natriumacetat eingesetzt wird.

6. Verfahren nach einem der Ansprüche 1 bis 4, wobei als Katalysator Natriummethacrylat eingesetzt wird.

7. Verfahren nach einem der vorstehenden Ansprüche, wobei der Katalysator in einer Menge von 0,2 bis 5,0 mol% eingesetzt wird.

8. Verfahren nach einem der vorstehenden Ansprüche, wobei die Umsetzung bei einer Temperatur im Bereich von 50 bis 120 °C erfolgt.

9. Verfahren nach einem der vorstehenden Ansprüche, wobei die Umsetzung über 1 bis 13 h erfolgt.

10. Verfahren nach einem der vorstehenden Ansprüche, wobei das Benzophenon(meth)acrylat in Wasser ausgefällt wird.

## Claims

1. Process for preparing benzophenone (meth)acrylates, **characterized in that** hydroxybenzophenone and (meth)acrylic anhydride are reacted in the presence of catalytic amounts of salt selected from the group consisting of sodium, lithium or potassium acetates and/or Li, Na or K (meth)acrylates, the amount-of-substance ratio of (meth)acrylic anhydride to hydroxybenzophenone being 1.0:1.0 to 1.6:1.0.

2. Process according to Claim 1, the amount-of-substance ratio being situated in the range from 1.05:1.0 to 1.5:1.0, preferably in the range from 1.1:1.0 to 1.4:1.0, more preferably in the range from 1.1:1.0 to 1.2:1.0.

3. Process according to Claim 1 or 2, where as hydroxybenzophenone 4-hydroxybenzophenone is used.

4. Process according to any of the preceding claims, where methacrylic anhydride or acrylic anhydride is used.

5. Process according to any of the preceding claims, where sodium acetate is used as catalyst.

6. Process according to any of Claims 1 to 4, where sodium methacrylate is used as catalyst.

7. Process according to any of the preceding claims, where the catalyst is used in an amount of 0.2 to 5.0 mol% .

8. Process according to any of the preceding claims, where the reaction takes place at a temperature in the range from 50 to 120°C.

9. Process according to any of the preceding claims, where the reaction takes place over 1 to 13 h.

10. Process according to any of the preceding claims, where the benzophenone (meth)acrylate is precipitated in water.

## Revendications

1. Procédé de fabrication de (méth)acrylates de benzophénone, **caractérisé en ce que** de l'hydroxybenzophénone et de l'anhydride de l'acide (méth)acrylique sont mis en réaction en présence de quantités catalytiques de sels, choisis dans le groupe constitué par les acétates de sodium, de lithium ou de potassium ou les (méth)acrylates de Li, Na ou K,
le rapport entre les quantités de matière d'anhydride de l'acide (méth)acrylique et d'hydroxybenzophénone étant de 1,0:1,0 à 1,6:1,0.

2. Procédé selon la revendication 1, dans lequel le rapport entre les quantités de matière se situe dans la plage allant de 1,05:1,0 à 1,5:1,0, de préférence dans la plage allant de 1,1:1,0 à 1,4:1,0, de manière davantage préférée dans la plage allant de 1,1:1,0 à 1,2:1,0.

3. Procédé selon la revendication 1 ou 2, dans lequel la 4-hydroxybenzophénone est utilisée en tant qu'hydroxybenzophénone.

4. Procédé selon l'une quelconque des revendications précédentes, dans lequel de l'anhydride de l'acide méthacrylique ou de l'anhydride de l'acide acrylique est utilisé.

5. Procédé selon l'une quelconque des revendications précédentes, dans lequel de l'acétate de sodium est utilisé en tant que catalyseur.

6. Procédé selon l'une quelconque des revendications 1 à 4, dans lequel du méthacrylate de sodium est utilisé en tant que catalyseur.

7. Procédé selon l'une quelconque des revendications précédentes, dans lequel le catalyseur est utilisé en une quantité de 0,2 à 5,0 % en moles.

8. Procédé selon l'une quelconque des revendications précédentes, dans lequel la réaction a lieu à une température dans la plage allant de 50 à 120 °C.

9. Procédé selon l'une quelconque des revendications précédentes, dans lequel la réaction a lieu pendant 1 à 13 h.

10. Procédé selon l'une quelconque des revendications précédentes, dans lequel le (méth)acrylate de benzophénone est précipité dans de l'eau.
